# EUROPEAN PATENT SPECIFICATION

(11) **EP 0 608 006 B1**
(45) Date of publication and mention of the grant of the patent: **10.03.1999**
(21) Application number: 94104814.2
(22) Date of filing: 31.05.1991
(51) Int. Cl.: G01N 21/07, B04B 5/04

(54) **Analytical rotors and methods for analysis of biological fluids**
Rotoren für die Analytik und Verfahren zur Analyse biologischer Fluide
Rotors analytiques et procédés d'analyse de fluides biologiques

(30) Priority: 04.06.1990 US 532524; 01.04.1991 US 678762; 01.04.1991 US 678823; 01.04.1991 US 678824
(43) Date of publication of application: 27.07.1994
(62) Divisional of application: 91910787.0
(73) Proprietor: ABAXIS, INC., Sunnyvale, California 94089 (US)
(72) Inventor: Burd,Tammy Leigh, Fremont, CA 94536 (US); Schembri,Carol T., San Matteo, CA 94403 (US); Braynin,Boris, Santa Clara, CA 95051 (US); Ostoich,Vladimir, Los Altos, CA 94022 (US)
(74) Representative: Sparing - Röhl - Henseler Patentanwälte

(56) References cited:
- EP-A- 0 039 825
- EP-A- 0 160 282
- FR-A- 2 575 293
- US-A- 3 744 975

## Description

The invention relates to a rotor for centrifuging biological samples including a liquid phase, comprising a bulk fluid chamber, a metering chamber connected to the bulk fluid chamber and being positioned radially outward from the bulk fluid chamber, an overflow chamber connected to the metering chamber, a receiving chamber positioned radially outward from the metering chamber, and a capillary connecting means for delivering the fluid from the metering chamber to the receiving chamber.

Further the invention relates to a method for delivering a predetermined volume of fluid to a receiving chamber in a rotor for centrifuging biological samples including a liquid phase, wherein a volume of fluid greater than the predetermined volume is introduced into a bulk fluid chamber, the rotor is spinned at a first rotational speed to effect the radially outward flow of the fluid from the bulk fluid chamber into a metering chamber such that excess fluid flows out of the metering chamber into an overflow chamber, and a predetermined volume of fluid remains in the metering chamber, and fluid from the metering chamber is delivered from the metering chamber to the receiving chamber.

Such a rotor and method are known from EP-A-0 039 825, a capillary connecting means for delivering the fluid from a metering chamber to a receiving chamber being provided by a straight capillary which is used to hold the fluid in the receiving chamber by capillary force and the friction of the meniscus of the fluid which hinders an escape of air from the receiving chamber. For transporting the fluid into the receiving chamber an increase of the rotational speed of the rotor is used to break the meniscus.

FR-A-2 575 293 concerns a rotor wherein the connecting means between the metering chamber and the receiving chamber are also provided as a capillar extending in circumferential direction. - US-A-3 744 975 describes a rotor wherein the connecting means is a folded passage which prevents direct passage of sample fluid to a receiving chamber after it has been distributed to the connecting means as the acceleration induced pressure head of the sample liquid in each connecting means is balanced by the pressure head of fluid in the inwardly extending leg of the connecting means.

It is desirable to provide an improved centrifugal rotor and method for separating blood into plasma and cellular components and for further distributing the separated plasma into a plurality of descrete test wells within the rotor.

Thus the invention concerns a rotor as mentioned above, wherein the connecting means comprises a siphon through which the metering chamber is capable of preventing flow of fluid out of the metering chamber until after the metering chamber is full, wherein the elbow of the siphon is positioned so that it is substantially the same distance from the centre of the rotor as the radially most inward point of the metering chamber.

The receiving chamber may be a separation chamber having a cell trap. A collection chamber may be connected to the receiving chamber and a plurality of cuvettes may be disposed radially outward from the collection chamber. Each cuvette may contain reagents necessary for analysis of the fluid. The rotor may be injection molded or machined.

Further, the invention concerns a method as mentioned above, wherein the fluid delivering from the metering chamber to the receiving chamber is practiced in stopping the rotation of the rotor, thereby priming a siphon connecting the metering chamber to the receiving chamber, followed by spinning the rotor, thereby initiating the operation of the siphon and emptying the metering chamber.

The receiving chamber may be used as a separation chamber and be provided with a cell trap. The bulk fluid chamber may be used as a diluent chamber and the introduction of fluid may be carried out by preloading a diluent in the bulk fluid chamber. As biological sample blood may be centrifuged.

The invention will now be explained in connection with the embodiments shown in the attached drawings.

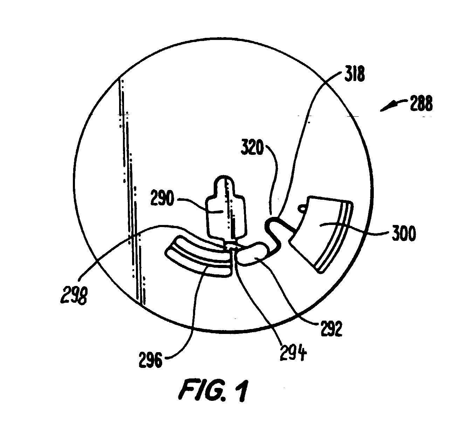

Fig. 1 is a plan view of a middle layer of a centrifugal rotor.

Fig. 2 is a plan view of a bottom layer of a centrifugal rotor.

Fig. 3 shows a part of the bottom layer of Fig. 2.

Fig. 4 shows two cross-sectional views along the line 27-27 of Fig. 3.

Fig. 5 is a cross-sectional view of along line 28-28 of Fig. 2.

Fig. 6 is a perspective view of an inlet channel showing the direction of flow in the discrete flow paths.

Fig. 7 is a cross-sectional view of the bottom layer of the centrifugal rotor showing the light path through the fluid in the cuvette.

Fig. 8 is a cross-sectional view along line 31-31 of Fig. 7.

Fig. 9 is a plan view of a part of the centrifugal rotor showing a cuvette, a straight inlet channel and a reflective surface.

The centrifugal rotor is in the form of a substantially solid disk including a top layer (not shown), middle layer 288, and bottom layer 322 laminated together to form a composite structure. Typically, each of the layers will be composed of the same material, usually a transparent plastic such as an acrylate, but it is possible that the layers will be composed of different materials and that each layer may include two or more different materials forming different portions of the layer. A receptacle is formed in the bottom layer 322 and is generally aligned with the vertical axis of the rotor. The receptacle is formed to mate with the drive shaft of a conventional centrifuge system.

The top layer includes a blood application port and four vent ports. The blood application port and vent ports penetrate the entire thickness of the top layer and are aligned with various chambers formed in the middle layer 288 of the rotor.

The middle layer 288 comprises a blood capillary 290 and a metering chamber 292 connected to the blood capillary 290 by a connecting channel 294. An overflow chamber 296 is connected to the metering chamber 292 through the overflow channel 298. The blood capillary 290, the metering chamber 292 and overflow chamber 296 preferably have capillary dimensions. An initial volume of fluid, such as whole blood, is introduced into the blood capillary 290 through blood application port in the top layer. As the rotor spins the initial volume partitions between the metering chamber 292 and the overflow chamber 296. The metering chamber 292 is sized to accept the predetermined amount of fluid desired to be split from the blood capillary 290. The first fluid entering the metering chamber 292 will fill the chamber, while excess fluid will overflow the metering chamber 292 and flow through connecting channel 294 and overflow channel 298 into overflow chamber 296. The overflow feature causes the original bulk amount of fluid to be split into two amounts, the first precisely measured amount and the excess fluid.

A siphon 318 is used as the connecting means to control flow between the metering chamber 292 and separation chamber 300. The elbow 320 of the siphon 318 is positioned so that it is substantially the same distance from the center of the rotor as the radially most inward point of the metering chamber 292.

As the rotor 10 spins and the metering chamber 292 is filled, fluid in the siphon 318 does not move past the elbow 320. The rotor 10 is then stopped and capillary action pulls fluid just beyond the elbow 320 and the siphon is "primed". When the rotor 10 is spun again, centrifugal and capillary forces pull the fluid out of the metering chamber 292 into the separation chamber 300.

The bottom layer 322 typically of a transparent plastic, such as acrylic, comprises a sample collection chamber 324 spaced radially inward from a plurality of peripheral cuvettes 326. Each cuvette 326 is connected to the collection chamber 324 by an inlet channel 328. The collection chamber may be formed in any shape, for instance, as a circle, a ring, or the like.

Each inlet channel 328 comprises two discrete flow paths, a first flow path 340 for the flow of liquid into the cuvette 326 and a second flow path 342 for the flow of gas out of the cuvette. The term "discrete" as used herein refers to the fact that two flow paths 340 and 342 are separately defined and distinct from each other. The inlet channels 328 are preferably curved so as to prevent backwash or carryover when the contents of the cuvettes are agitated to effect mixing of the contents. Thus, cross contamination between cuvettes is avoided. The use of the flow paths 340 and 342 allow gas to escape easily from the cuvette 326 as it is filled and thus prevent the formation of bubbles in the cuvette 326, which can deleteriously affect the results of optical analyses.

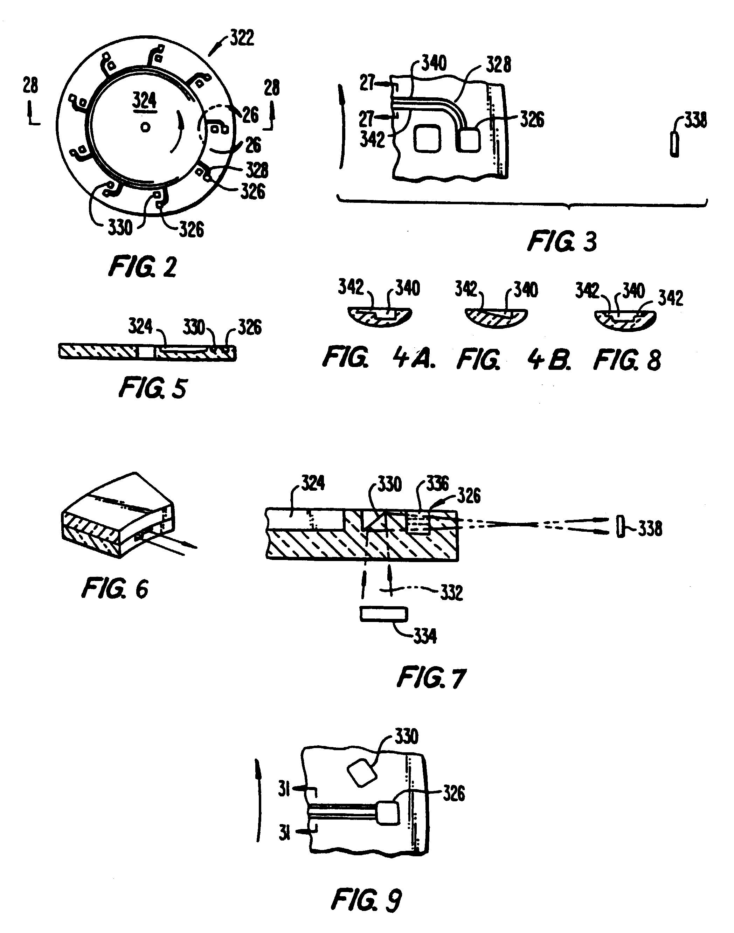

There are a number of ways to create two discrete flow paths in the inlet channel 328. For instance, Figs. 4A, 4B and 8 show three possible configurations in which liquid flow path 340 has a greater depth than the gas flow path 342. Because of its greater depth, the fluid will preferentially flow down path 340, leaving path 342 available for the evacuation of gas from the cuvette 328. The liquid flow path 340 may be on the side of the inlet channel 328 toward the direction of rotation of the rotor, as shown in Figs. 4A and 4B. In this configuration, centrifugal force will urge the liquid along the "leading" wall. Alternatively, if the inlet channel is not curved as shown in Fig. 9, the fluid flow path 340 may be in the center of the inlet channel 328, as shown in Fig. 8.

The inlet channel 328 is conveniently formed such that it passes around a reflective surface 330 (described more fully, below). If a reflective surface 330 is present, the inlet channel 328 will typically pass around the reflective surface 330 on the side in the direction of rotation of the rotor. In the absence of a reflective surface 330, the inlet channel may be formed in any other generally radial configuration.

Other embodiments utilize inlet channels 328 having regions with different surface textures. For instance, the gas flow path 342 may be left unpolished, leaving a rough surface texture in that region, while the fluid flow path 340 is polished. Alternatively, the liquid flow path 340 may be treated so as to be hydrophilic whereas the gas flow path is treated so as to be hydrophobic. The manner of treatment to make the surfaces hydrophilic or hydrophobic is well known in the art and need not be recited here. Any known surface treatment may be used as desired so long as it is chemically inert to the fluids passing through the inlet channel 328.

The rotor thus permits rapid filling of the cuvettes. Each cuvette is filled completely leaving little or no gas to interfere with subsequent optical analysis of the cuvettes contents.

Turning now to Fig. 7, it can be seen that optical analysis of the cuvette contents is facilitated by reflective surfaces 330 positioned radially inward from each cuvette 326 such that they are capable of deflecting a light beam between a generally vertical and a generally horizontal direction and which are oriented at about 45° from the vertical axis of the rotor. As used herein, the "horizontal" and "vertical" directions are determined in relation to the axis of rotation of the rotor. The horizontal direction (typically radial) is perpendicular to the axis and the vertical direction is parallel to the axis.

The reflective surface 330 need not be oriented directly radially inward from the cuvette. The reflective surface 330, however, must be parallel to the side of the cuvette in the optical pathway. For instance, a horizontal light beam which does not pass radially through the rotor may be used. Thus, the reflective surface 330 will be placed on a radial plane different from that of the cuvette 326, as shown in Fig. 9.

In an exemplified embodiment, the reflective surface deflects a vertical light beam 332 from a light source 334 so that it passes radially through a fluid 336 in the cuvette 326. A light is then detected by the detector 338. The orientation of the reflective surfaces 330 is such that the positions of the detector 338 and light source 334 can be reversed. In the reversed configuration a horizontal light beam passes through the cuvette contents and is then deflected so that it passes vertically through the rotor where it is detected below the rotor. The reflective surfaces can be composed of any reflective surface known in the art which provides total internal reflection, and are typically air mirrors in which light is reflected at the acrylic-air interface. Alternatively, the surface can be coated or backed with a light reflective material.

## Claims

1. A rotor for centrifuging biological samples including a liquid phase, comprising a bulk fluid chamber (304), a metering chamber (292) connected to the bulk fluid chamber (304) and being positioned radially outward from the bulk fluid chamber (304), an overflow chamber (296) connected to the metering chamber (292), a receiving chamber (300) positioned radially outward from the metering chamber (292), and a capillary connecting means for delivering the fluid from the metering chamber (292) to the receiving chamber (300), **characterized** in that the connecting means comprises a siphon (318) through which the metering chamber (292) is capable of preventing flow of fluid out of the metering chamber (292) until after the metering chamber (292) is full, wherein the elbow (320) of the siphon (318) is positioned so that it is substantially the same distance from the centre of the rotor as the radially most inward point of the metering chamber (292).

2. The rotor of claim 1, characterized in that the receiving chamber (300) is a separation chamber having a cell trap.

3. The rotor of claim 1 or 2, characterized in that a collection chamber (324) connected to the receiving chamber (300) and a plurality of cuvettes (326) disposed radially outward from the collection chamber (324) are provided.

4. The rotor of claim 3, characterized in that each cuvette (326) contains reagents necessary for analysis of the fluid.

5. The rotor of anyone of the claims 1 to 4, characterized in that it is injection molded or machined.

6. A method for delivering a predetermined volume of fluid to a receiving chamber (300) in a rotor for centrifuging biological samples including a liquid phase, wherein a volume of fluid greater than the predetermined volume is introduced into a bulk fluid chamber (304), the rotor is spinned at a first rotational speed to effect the radially outward flow of the fluid from the bulk fluid chamber (304) into a metering chamber (292) such that excess fluid flows out of the metering chamber (292) into an overflow chamber (296), and a predetermined volume of fluid remains in the metering chamber (292), and fluid from the metering chamber (292) is delivered from the metering chamber (292) to the receiving chamber (300), **characterized** in that the fluid delivering from the metering chamber (292) to the receiving chamber (300) is practiced in stopping the rotation of the rotor, thereby priming a siphon (318) connecting the metering chamber (292) to the receiving chamber (300), followed by spinning the rotor, thereby initiating the operation of the siphon (318) and emptying the metering chamber (292).

7. The method of claim 6, characterized in that the receiving chamber (300) is used as an separation chamber and is provided with a cell trap.

8. The method of claim 6 or 7, characterized in that the bulk fluid chamber (304) is used as an diluent chamber and the introduction of fluid is carried out by preloading a diluent in the bulk fluid chamber (304).

9. The method of anyone of the claims 6 to 8, characterized in that as biological sample blood is centrifuged.

## Patentansprüche

1. Rotor zum Zentrifugieren von biologischen Proben, die eine Flüssigphase einschließen, umfassend eine Flüssigkeitsmassenkammer (304), eine Bemessungskammer (292), die mit der Flüssigkeitsmassenkammer (304) verbunden und radial auswärts von der Flüssigkeitsmassenkammer (304) angeordnet ist, eine Überlaufkammer (296), die mit der Bemessungskammer (292) verbunden ist, eine Aufnahmekammer (300), die radial auswärts von der Bemessungskammer (292) angeordnet ist, und ein kapillares Verbindungsmittel zum Liefern von Flüssigkeit von der Bemessungskammer (292) zur Aufnahmekammer (300), **dadurch gekennzeichnet,** daß die Verbindungsmittel einen Siphon (318) umfassen, durch den die Bemessungskammer (292) in der Lage ist, das Fließen von Flüssigkeit aus der Bemessungskammer (292) zu verhindern, bis die Bemessungskammer (292) gefüllt ist, wobei das Knie (320) des Siphons (318) derart positioniert ist, daß es sich im wesentlichen im gleichen Abstand vom Mittelpunkt des Rotors wie die radial am meisten einwärts gelegene Stelle der Bemessungskammer (292) befindet.

2. Rotor nach Anspruch 1, dadurch gekennzeichnet, daß die Aufnahmekammer (300) eine Trennkammer ist, die eine Zellenfalle besitzt.

3. Rotor nach Anspruch 1 oder 2, dadurch gekennzeichnet, daß eine Sammelkammer (324) mit der Aufnahmekammer (300) verbunden ist und eine Vielzahl von Küvetten (326) radial auswärts von der Sammelkammer (324) vorgesehen ist.

4. Rotor nach Anspruch 3, dadurch gekennzeichnet, daß jede Küvette (326) Reagenzien enthält, die zur Analyse der Flüssigkeit notwendig sind.

5. Rotor nach einem der Ansprüche 1 bis 4, dadurch gekennzeichnet, daß er spritzgegossen oder spanend bearbeitet ist.

6. Verfahren zum Abgeben eines vorbestimmten Flüssigkeitsvolumens an eine Aufnahmekammer (300) in einem Rotor zum Zentrifugieren von biologischen Proben, die eine Flüssigphase einschließen, wobei ein Flüssigkeitsvolumen größer als das vorbestimmte Volumen in eine Flüssigkeitsmassenkammer (304) eingeführt, der Rotor mit einer ersten Drehgeschwindigkeit gedreht wird, um den radial auswärtigen Fluß der Flüssigkeit aus der Flüssigkeitsmassenkammer (304) in eine Bemessungskammer (292) zu bewirken, so daß überschüssige Flüssigkeit aus der Bemessungskammer (292) in eine Überlaufkammer (296) fließt, und ein vorbestimmtes Flüssigkeitsvolumen in der Bemessungskammer (292) bleibt, und Flüssigkeit aus der Bemessungskammer (292) aus der Bemessungskammer (292) in die Aufnahmekammer (300) geliefert wird, **dadurch gekennzeichnet**, daß die Flüssigkeitsabgabe von der Bemessungskammer (292) zur Aufnahmekammer (300) durch Anhalten der Drehung des Rotors vorgenommen wird, wodurch ein Siphon (318), der die Bemessungskammer (292) mit der Aufnahmekammer (300) verbindet, gefüllt wird, gefolgt von einem Drehen des Rotors, um den Betrieb des Siphons (318) in Gang zu setzen und die Bemessungskammer (292) zu leeren.

7. Verfahren nach Anspruch 6, dadurch gekennzeichnet, daß die Aufnahmekammer (300) als eine Trennkammer verwendet wird und mit einer Zellenfalle versehen ist.

8. Verfahren nach Anspruch 6 oder 7, dadurch gekennzeichnet, daß die Flüssigkeitsmassenkammer (304) als eine Verdünnungskammer verwendet wird und das Einführen der Flüssigkeit unter Voreinfüllen eines Verdünnungsmittels in die Flüssigkeitsmassenkammer (304) ausgeführt wird.

9. Verfahren nach einem der Ansprüche 6 bis 8, dadurch gekennzeichnet, daß als biologische Probe Blut zentrifugiert wird.

## Revendications

1. Rotor pour centrifuger des échantillons biologiques comprenant une phase liquide, comportant une chambre à liquide en vrac (304), une chambre de mesure (292) raccordée à la chambre à liquide en vrac (304) et disposée radialement à l'extérieur par rapport à la chambre à liquide en vrac (304), une chambre de débordement (296) raccordée à la chambre de mesure (292), une chambre réceptrice (300) disposée radialement vers l'extérieur par rapport à la chambre de mesure (292), et un moyen de raccordement capillaire pour délivrer le liquide de la chambre de mesure (292) à la chambre réceptrice (300), caractérisée en ce que le moyen de raccordement comporte un siphon (318) par lequel la chambre de mesure (292) est capable d'empêcher le liquide de s'écouler de la chambre de mesure (292) jusqu'après que la chambre de mesure (292) soit pleine, le coude (320) du siphon (318) étant placé de telle sorte qu'il est sensiblement à la même distance du centre du rotor que le point radialement le plus intérieur de la chambre de mesure (292).

2. Rotor selon la revendication 1, caractérisé en ce que la chambre réceptrice (300) est une chambre de séparation ayant un piège à cellules.

3. Rotor selon la revendication 1 ou 2, caractérisé en ce qu'il est prévu une chambre collectrice (324) raccordée à la chambre réceptrice (300) et une pluralité de cuvettes (326) disposées radialement vers l'extérieur par rapport à la chambre collectrice (324).

4. Rotor selon la revendication 3, caractérisé en ce que chaque cuvette (326) contient des réactifs nécessaires pour l'analyse du liquide.

5. Rotor selon l'une quelconque des revendications 1 à 4, caractérisé en ce qu'il est moulé par injection ou usiné.

6. Procédé pour délivrer un volume prédéterminé de liquide à une chambre réceptrice (300) dans un rotor pour la centrifugation d'échantillons biologiques comprenant une phase liquide, dans lequel un volume de liquide supérieur au volume prédéterminé est introduit dans une chambre à liquide en vrac (304), le rotor est mis en rotation à une première vitesse de rotation pour faire couler le liquide radialement en direction de l'extérieur, de la chambre à liquide en vrac (304) à une chambre de mesure (292) de façon que le liquide en excès s'écoule de la chambre de mesure (292) à une chambre de débordement (296) et qu'un volume prédéterminé de liquide reste dans la chambre de mesure (292), et du liquide de la chambre de mesure (292) est délivré de la chambre de la chambre de mesure (292) à la chambre réceptrice (300), caractérisé en ce que la délivrance de liquide de la chambre de mesure (292) à la chambre réceptrice (300) est pratiquée en arrêtant de la rotation du rotor, ceci amorçant un siphon (318) raccordant la chambre de mesure (292) à la chambre réceptrice (300), et ensuite en faisant tourner le rotor, ceci faisant commencer le fonctionnement du siphon (318) et vider la chambre de mesure (292).

7. Procédé selon la revendication 6, caractérisé en ce que la chambre réceptrice (300) est utilisée comme chambre de séparation et comporte un piège à cellules.

8. Procédé selon la revendication 6 ou 7, caractérisé en ce que la chambre à liquide en vrac (304) est utilisée comme chambre à diluant et en ce que l'introduction de liquide est effectuée en chargeant au préalable un diluant dans la chambre à liquide en vrac (304).

9. Procédé selon l'une quelconque des revendications 6 à 8, caractérisé en ce que du sang est centrifugé en tant qu'échantillon biologique.
